# EUROPEAN PATENT APPLICATION

(11) **EP 4 681 706 A1**
(43) Date of publication of application: **21.01.2026**
(21) Application number: 24778069.5
(22) Date of filing: 27.03.2024
(51) Int. Cl.: A61K 9/72, A61M 11/00, A61M 15/00, A61K 9/08, A61K 47/10, A61K 31/137, A61P 11/10, A61P 11/00

(54) **LIQUID PREPARATION ALLOWING FOR NEBULIZATION INHALATION, CARTRIDGE, AND AEROSOL GENERATION SYSTEM**

(30) Priority: 31.03.2023 CN 202310361703
(71) Applicant: Shenzhen First Union Technology Co., Ltd., Shenzhen, Guangdong 518000 (CN)
(72) Inventor: LU, Jin, Shenzhen, Guangdong 518000 (CN); ZHAO, Jing, Shenzhen, Guangdong 518000 (CN); DENG, Jingjing, Shenzhen, Guangdong 518000 (CN); LIU, Zhang, Shenzhen, Guangdong 518000 (CN); CHU, Ming, Shenzhen, Guangdong 518000 (CN); HUANG, Fuyuan, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2024/084101
(87) International publication number: WO 2024/199283

(57) **Abstract**

Embodiments of the present application disclose a liquid preparation, a cartridge for an aerosol generation system, and an aerosol generation system. The liquid preparation comprises a solvent and ambroxol hydrochloride. The atomization conversion rate of ambroxol hydrochloride in the liquid preparation to form aerosol after being heated and atomized by an electronic atomizer is not lower than 90%; or the atomization conversion rate of ambroxol hydrochloride is not lower than 80%; or the atomization conversion rate of ambroxol hydrochloride is not lower than 70%; or the atomization conversion rate of ambroxol hydrochloride is not lower than 60%.

## Description

The present application claims priority to Chinese Patent Application No. 202310361703.6, filed with the China National Intellectual Property Administration on March 31, 2023 and entitled "LIQUID PREPARATION FOR ATOMIZATION INHALATION, CARTRIDGE, AND AEROSOL GENERATING SYSTEM", which is incorporated herein by reference in its entirety.

### TECHNICAL FIELD

Embodiments of the present application relate to the field of aerosol generating devices, and in particular relates to a liquid preparation for atomization inhalation, a cartridge, and an aerosol generating system.

### BACKGROUND

Ambroxol hydrochloride (AMB) is a semisynthetic derivative of vasicine extracted from the Indian shrub Adhatoda vasica. Ambroxol hydrochloride is a new active metabolite of bromhexine *in vivo,* has high safety and few side effects, can be used in children, and is widely used in clinical practice. Ambroxol hydrochloride is mainly used to promote elimination of viscous secretions from the respiratory tract and reduce mucus retention, thus significantly promoting sputum discharge, and being suitable for acute or chronic respiratory disease with abnormal sputum secretion and poor sputum discharge function.

AMB injection is commonly used for slow intravenous injection therapy in clinical practice.

Chinese patent CN110179772A discloses administration of an ambroxol hydrochloride solution for inhalation by means of a modern medical aerosol device (e.g., a compressed air or oxygen atomizer, an ultrasonic atomizer, and a mesh vibrating atomizer), and the medical nebulizing device is usually not portable.

### SUMMARY

Embodiments of the present application provide a liquid preparation for atomization inhalation, used for an electronic atomizer, and including:
a solvent; and
ambroxol hydrochloride, where
when the ambroxol hydrochloride in the liquid preparation is heated and atomized by the electronic atomizer to generate an aerosol, the atomization conversion rate of the ambroxol hydrochloride is not lower than 90%;
or the atomization conversion rate of the ambroxol hydrochloride is not lower than 80%;
or the atomization conversion rate of the ambroxol hydrochloride is not lower than 70%;
or the atomization conversion rate of the ambroxol hydrochloride is not lower than 60%.

In the above embodiments, ambroxol hydrochloride is prepared into a liquid preparation for atomization inhalation, and the liquid preparation is added to an electronic atomizer and atomized by electric heating to generate an aerosol. On the one hand, currently electronic atomization devices are all portable and convenient for users to carry, which increases the patient compliance and enriches the application scenarios of clinical treatment. On the other hand, the boiling point of ambroxol hydrochloride is about 468°C, the working temperature range of the current electronic atomizers is roughly 100-500°C, so that ambroxol hydrochloride can incur azeotropy with commonly used solvents in the electronic atomizers, thereby generating an aerosol.

Further, by preparing ambroxol hydrochloride into a liquid preparation and heating and atomizing the liquid preparation by an electronic atomization device to test for convertibility of the liquid preparation into an aerosol, it is found through the heating and atomization testing that ambroxol hydrochloride has a high atomization conversion rate. By the atomization testing method according to the embodiments of the present application, it is found that the liquid preparation containing a certain amount of ambroxol hydrochloride has a atomization conversion rate of not less than 90%, and the mass percentage content is roughly set to be 0.1-1%. When the content of the ambroxol hydrochloride in the liquid preparation is adjusted to be in a certain range, the conversion rate of the ambroxol hydrochloride is not less than 80%. When the content of the ambroxol hydrochloride in the liquid preparation is further adjusted to be in other ranges, or a different electronic atomizer is used, the conversion rate of the ambroxol hydrochloride is not less than 70%, or the conversion rate of the ambroxol hydrochloride is not less than 60%. The different electronic atomizer is configured with a different heating power or uses a different atomization core component.

In such a case, when a user inhales the aerosol generated by atomizing the liquid preparation, the ambroxol hydrochloride is inhaled through the lungs and reaches the alveoli, and can be quickly absorbed and enter the blood circulation to achieve high bioavailability. Therefore, by preparing the ambroxol hydrochloride into the liquid preparation and atomizing and inhaling the liquid preparation of ambroxol hydrochloride, the therapeutic effect of the ambroxol hydrochloride can be fully utilized.

Compared to an ultrasonic atomizer which generates water mist by breaking up the molecular structure of a liquid substrate through high-frequency oscillation of a mesh atomizing plate, an electronic atomizer brings a liquid substrate to come to the boiling point and generate an aerosol through an electric heating process. The particle size of most of the aerosol generated by the liquid preparation containing ambroxol hydrochloride through the electric heating process using the electronic atomizer is less than or equal to 1 µm, which is conducive to deep deposition of the aerosol in the bronchi and lungs, especially in the deep alveoli, and is suitable for drugs for administration in the lower respiratory tract and lungs. Combining the significant therapeutic effect of the ambroxol hydrochloride in the treatment of respiratory disease, the liquid preparation containing the ambroxol hydrochloride being atomized through electric heating using the electronic atomizer is more conducive to utilization of the therapeutic effect of the ambroxol hydrochloride. On the other hand, by atomizing the liquid preparation containing the ambroxol hydrochloride using the electronic atomizer, the generated ambroxol hydrochloride aerosol has less residue in the oral cavity and less irritation to the stomach, which is beneficial for improving user compliance. Further, the electronic atomizer is configured to an active inhalation mode, which activates a heating system through a user's active inhalation, and then the aerosol containing ambroxol hydrochloride generated by atomization through electric heating is inhaled, which can further enhance the user compliance.

In some embodiments, the weight percentage of the ambroxol hydrochloride in the liquid preparation is 0.01-2%; or the weight percentage of the ambroxol hydrochloride in the liquid preparation is 0.1-1%.

Ambroxol hydrochloride is administered through intravenous drip, with a daily dosage limit of no more than 45 mg for adults and up to 90 mg for severe cases. Therefore, by combining the capacity of an aerosol in each puff of the electronic atomizer and the number of inhalations of a user per day, the content of ambroxol hydrochloride is controlled within 2%, to allow the user to inhale multiple times within a day to alleviate lung discomfort symptoms such as pneumonia and asthma.

Further, by testing the atomization effect of the liquid preparation of ambroxol hydrochloride, it is found that when the weight percentage of the ambroxol hydrochloride in the liquid preparation is controlled at around 1%, the conversion rate of the ambroxol hydrochloride in the liquid preparation into the aerosol is substantially 90% or higher, even close to 100%. Therefore, controlling the weight percentage of the ambroxol hydrochloride in the liquid preparation within 1% is beneficial for improving the conversion rate of the ambroxol hydrochloride.

The above ambroxol hydrochloride is a pharmaceutical grade raw material that may be prepared by methods reported in the related art.

In some embodiments, the solvent includes at least one of propylene glycol, vegetable glycerin, water, or ethanol.

In some embodiments, the weight percentage of the propylene glycol in the liquid preparation is 20-80%. Further, the specific mass percentage of the propylene glycol in the liquid preparation may be any quantity value selected from a range of 20-80%.

In some embodiments, the weight percentage of the vegetable glycerin in the liquid preparation is 30-70%. Further, the specific mass percentage of the vegetable glycerin in the liquid preparation may be any quantity value selected from a range of 30-70%.

In some embodiments, the weight percentage of the water in the liquid preparation is 10-50%. Further, the specific mass percentage of the water in the liquid preparation may be any quantity value selected from a range of 10-50%.

The above propylene glycol may be 1,2-propylene glycol or 1,3-propylene glycol.

Propylene glycol and vegetable glycerin, as common solvents in electronic atomizers, have excellent performance in increasing the aerosol amount and controlling the fluidity of a liquid preparation. Therefore, propylene glycol and vegetable glycerin are the main solvent components. Also, an appropriate amount of water or ethanol may be added as a solvent as the weight percentage of the ambroxol hydrochloride in the liquid preparation increases, thereby increasing the solubility of the ambroxol hydrochloride in the liquid preparation.

The content of the above water in the liquid preparation needs to be controlled within an appropriate range. Adding water to the solvent is beneficial for improving the solubility of the ambroxol hydrochloride. However, an increase in the water content in the solvent will increase the risk of leakage and the risk of a user inhaling excessive condensate when the solvent is added to the electronic atomizer. Therefore, the water content needs to be controlled within the above range.

In some embodiments, the particle size of the aerosol generated by atomizing the ambroxol hydrochloride is in a range of 0.2-3 µm.

In some embodiments, the liquid preparation further includes at least one of an edible essence, a sweetener, or a cooling agent.

The above edible essence is used to increase the taste and aromatic flavor of the aerosol generated by atomizing the liquid preparation. The types of flavors of the edible essence include one or more of fruit flavor, flower flavor, mint flavor, and tea flavor.

The weight percentage of the above edible essence in the liquid preparation is in a range of roughly 0-10%, and the specific content may be set to be any value in the range of 0-10% according to the taste adjustment needs of the liquid preparation. For example, the specific weight percentage of the edible essence is 5%.

The above sweetener is used to enhance the sweetness of the above aerosol generated by atomizing the liquid preparation. It should be noted that the ambroxol hydrochloride has a certain bitterness, so adding an appropriate amount of sweetener to the liquid preparation can help reduce the bitterness of the ambroxol hydrochloride and greatly enhance the pleasure of a user inhaling the aerosol containing the ambroxol hydrochloride. The sweetener may be selected from the group consisting of one or more of cyclamate, xylitol, mogroside, acesulfame K, saccharin sodium, sucralose, aspartame, alitame, neohesperidin dihydrochalcone, neotame, and stevioside.

The weight percentage of the above sweetener in the liquid preparation is in a range of roughly 0-10%, and the specific content may be set to be any value in the range of 0-10% according to the taste adjustment needs of the liquid preparation. For example, the specific weight percentage of the sweetener is 1%.

The above cooling agent is used to increase the cooling effect of the above aerosol generated by atomizing the liquid preparation, thereby enhancing a user's pleasure in inhaling the aerosol. Suitable cooling agents for use in the above liquid preparation may be one or more of menthol, menthone, isomenthone, WS-23 (2-isopropyl-N,2,3-trimethylbutanamide), WS-3 (N-ethyl-p-menthyl-3-carboxamide), and WS-5 (N-(ethoxycarbonylmethyl)-p-alkyl-3-carboxamide).

The weight percentage of the above cooling agent in the liquid preparation is in a range of roughly 0-10%, and the specific content may be set to be any value in the range of 0-10% according to the taste adjustment needs of the liquid preparation. For example, the specific weight percentage of the cooling agent is 2%.

The embodiments of the present application further provide a cartridge, used for an electronic atomizer, and the cartridge stores the above liquid preparation.

The above cartridge may be provided as a closed container that may be sold independently and assembled on an electronic atomizer. The liquid preparation in the cartridge may flow towards a atomization core component on the electronic atomizer and be atomized by the atomization core component to generate an aerosol.

The above cartridge may also be provided as an inseparable part of the electronic atomizer. The liquid preparation in the cartridge may flow towards a atomization core component on the electronic atomizer and be atomized by the atomization core component to generate an aerosol. As the liquid preparation in the cartridge is consumed, at least a part of the electronic atomizer may be discarded.

The embodiments of the present application further provide an aerosol generating system, including the above liquid preparation and an electronic atomizer for atomizing the liquid preparation to generate an aerosol.

In some embodiments, the electronic atomizer includes a atomizer using a ceramic core or a atomizer using a cotton core.

### BRIEF DESCRIPTION OF THE DRAWINGS

In order to more clearly illustrate the technical solutions of the embodiments of the present disclosure, a brief introduction is given below to the accompanying drawings required for use in the embodiments of the present disclosure. It is apparent that the drawings described below are only some embodiments of the present disclosure, and for those of ordinary skills in the art, other drawings can be obtained based on these drawings without creative effort.
FIG. 1 shows an HPLC chromatogram of a liquid preparation of ambroxol hydrochloride with a content of 0.5% according to some embodiments of the present application;
FIG. 2 shows an HPLC chromatogram of an aerosol generated by atomizing the liquid preparation of ambroxol hydrochloride with a content of 0.5% according to some embodiments of the present application;
FIG. 3 shows an HPLC chromatogram of a liquid preparation of ambroxol hydrochloride with a content of 1% according to some embodiments of the present application;
FIG. 4 shows an HPLC chromatogram of an aerosol generated by atomizing the liquid preparation of ambroxol hydrochloride with a content of 1% according to some embodiments of the present application; and
FIG. 5 shows a diagram of the particle size distribution of the aerosol of ambroxol hydrochloride according to some embodiments of the present application.

### DETAILED DESCRIPTION

For ease of understanding of the present application, the present application is described below in more detail with reference to specific implementations. The embodiments are only intended to provide a clearer understanding of the technical features, objectives, and effects of the present application, and are not intended to limit the present application.

The experimental methods used in the following embodiments are conventional methods unless otherwise specified.

The materials, instruments, and the like used in the following embodiments are commercially available unless otherwise specified.

In Embodiment I of the present application, ambroxol hydrochloride is prepared into a liquid preparation for atomization inhalation, which is used to generate an aerosol containing ambroxol hydrochloride through atomization using an electronic atomizer. The stability of the substance before and after atomization of the ambroxol hydrochloride is analyzed by a high-performance liquid chromatograph.

The materials and instruments used in Embodiment I are listed as follows:
Materials: ambroxol hydrochloride (AMB), 1,2-propylene glycol, vegetable glycerin, diammonium hydrogen phosphate, phosphoric acid, and acetonitrile.

Instruments: Shimadzu LC-20A high-performance liquid chromatograph from Japan, Labsolutions workstation, Cerulean smoking machine, and Sartorius BSA224S electronic balance.

### 1. Preparation of liquid preparation of ambroxol hydrochloride

The weight percentages of the components in the liquid preparation are: ambroxol hydrochloride: 0.5%; vegetable glycerin: 50%; water: 29.5%; and 1,2-propylene glycol: 20%.

### 2. Collection of aerosol generated by atomizing ambroxol hydrochloride

An appropriate amount of the liquid preparation was added to an electronic atomizer, a Cerulean smoking machine was used for suction, and the aerosol was collected using a single Cambridge filter. The parameters of the smoking machine used are: suction capacity: 55 mL; suction frequency: 30 s; suction duration: 3 s; and times of suction: 50. The aerosol collected on the Cambridge filter was collected and extracted by oscillation using an initial mobile phase.

### 3. Chromatographic condition

Thermo Fisher Scientific Acclaim 120 C18 (4.6*250 mm, 5 µm) chromatographic column was used. The Method for Measuring Substance Related to Ambroxol Hydrochloride in the 2020 edition *Chinese Pharmacopoeia* was referred to, with a mobile phase: acetonitrile/diammonium hydrogen phosphate solution (0.01 mol/L) (adjusted to pH 7.0 with phosphoric acid)=50:50; flow rate: 1.0 mL/min; detection wavelength: 248 nm; and column temperature: 30°C.

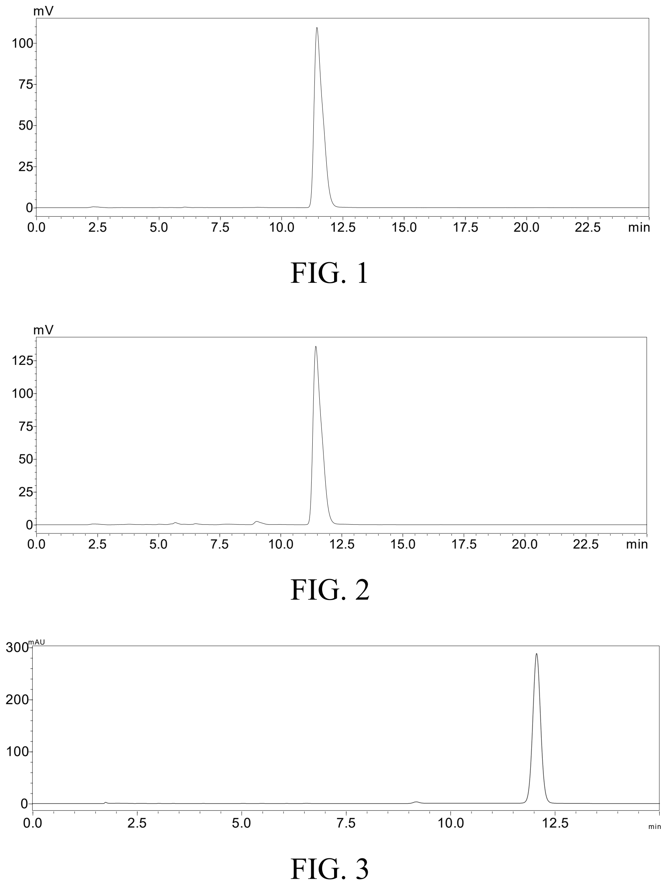

FIG. 1 shows a chromatogram of the liquid preparation of ambroxol hydrochloride, and FIG. 2 shows a chromatogram of the aerosol generated by atomizing the ambroxol hydrochloride. Comparing FIG. 1 and FIG. 2, it can be seen that there is no significant change in the peak shape of the chromatographic peak of the atomized ambroxol hydrochloride, and there are substantially no excess peaks in FIG. 2 compared to FIG. 1, indicating that the composition of the ambroxol hydrochloride is stable after the ambroxol hydrochloride is electrically heated and atomized by the electronic atomizer, and the ambroxol hydrochloride may not undergo cracking of substances due to atomization which may affect the efficacy.

Embodiment II of the present application takes a liquid preparation of ambroxol hydrochloride with a weight percentage of 0.5% as an example to calculate the conversion rate of ambroxol hydrochloride atomized to generate an aerosol.

The materials and instruments used in Embodiment II are as follows:
Materials: ambroxol hydrochloride (AMB), 1,2-propylene glycol, vegetable glycerin, diammonium hydrogen phosphate, phosphoric acid, and acetonitrile.

Instruments: Shimadzu LC-20A high-performance liquid chromatograph from Japan, Labsolutions workstation, Cerulean smoking machine, and Sartorius BSA224S electronic balance.

### 1. Preparation of liquid preparation of ambroxol hydrochloride

The weight percentages of the components in the liquid preparation are: ambroxol hydrochloride: 0.5%; vegetable glycerin: 50%; water: 29.5%; and 1,2-propylene glycol: 20%.

### 2. Collection of aerosol generated by atomizing ambroxol hydrochloride

An appropriate amount of the liquid preparation was added to an electronic atomizer, a Cerulean smoking machine was used for suction, and the aerosol was collected using a single Cambridge filter. The parameters of the smoking machine used are: suction capacity: 55 mL; suction frequency: 30 s; suction duration: 3 s; and times of suction: 50. The aerosol collected on the Cambridge filter was collected and extracted by oscillation using an initial mobile phase.

### 3. Chromatographic condition of high performance liquid chromatograph used

Thermo Fisher Scientific Acclaim 120 C18 (4.6*250 mm, 5 µm) chromatographic column was used. The Method for Measuring Substance Related to Ambroxol Hydrochloride in the 2020 edition *Chinese Pharmacopoeia* was referred to, with a mobile phase: acetonitrile : 0.01 mol/L diammonium hydrogen phosphate solution (adjusted to pH 7.0 with phosphoric acid) = 50:50; flow rate: 1.0 mL/min; detection wavelength: 248 nm; and column temperature: 30°C.

### 4. Test of conversion rate of ambroxol hydrochloride atomized to generate aerosol

### 4.1 Preparation of solution in blank control group and liquid preparation of ambroxol hydrochloride, and collection of ambroxol hydrochloride aerosol

Blank control: initial mobile phase.

Liquid preparation of ambroxol hydrochloride: an appropriate amount of liquid preparation was taken and diluted with the initial mobile phase.

Extraction of ambroxol hydrochloride aerosol: a Cambridge filter used during aerosol collection was taken and extracted by oscillation using the initial mobile phase.

### 4.2 Calculation method

The atomization conversion rate was calculated using a formula: A=w1/w2*100%
A=atomization conversion rate
w1=mass percentage of the ambroxol hydrochloride in the aerosol
w2=mass percentage of the ambroxol hydrochloride in the liquid preparation

The results of the test of the atomization conversion rate of the liquid preparation of ambroxol hydrochloride with a weight percentage of 0.5% are shown in Table 1 below.

**Table 1 Test results of atomization of 0.5% ambroxol hydrochloride**

| Sample | Weight of aerosol /g | Concentration of sample/g·mL⁻¹ | w/% | A/% |
|---|---|---|---|---|
| Aerosol | 0.267 | 0.043 | 0.494 | 98.21 |
| Liquid preparation | | 0.052 | 0.503 | |

| | | | | |
|---|---|---|---|---|
| Note: w represents the mass percentage of the ambroxol hydrochloride in the aerosol/liquid preparation; and A represents the atomization conversion rate. | | | | |

FIG. 1 shows a chromatogram of the liquid preparation of ambroxol hydrochloride with a weight percentage of 0.5%, and FIG. 2 shows a chromatogram of the aerosol generated by atomizing the liquid preparation of ambroxol hydrochloride. The atomization conversion rate of the 0.5% ambroxol hydrochloride measured by the above calculation method is 98.21%. Therefore, the composition of the ambroxol hydrochloride in the liquid preparation converted into the aerosol is stable and the conversion efficiency is high.

Embodiment III of the present application takes a liquid preparation of ambroxol hydrochloride with a mass percentage of 1% as an example to calculate the conversion rate of ambroxol hydrochloride atomized to generate an aerosol.

The materials and instruments used in Embodiment III are as follows:
Materials: ambroxol hydrochloride (AMB), 1,2-propylene glycol, vegetable glycerin, diammonium hydrogen phosphate, phosphoric acid, and acetonitrile.

Instruments: Shimadzu LC-20A high-performance liquid chromatograph from Japan, Labsolutions workstation, Cerulean smoking machine, and Sartorius BSA224S electronic balance.

### 1. Preparation of liquid preparation of ambroxol hydrochloride

The weight percentages of the components in the liquid preparation are: ambroxol hydrochloride: 1%; vegetable glycerin: 50%; water: 29%; and 1,2-propylene glycol: 20%.

### 2. Collection of aerosol generated by atomizing ambroxol hydrochloride

An appropriate amount of the liquid preparation was added to an electronic atomizer, a Cerulean smoking machine was used for suction, and the aerosol was collected using a single Cambridge filter. The parameters of the smoking machine used are: suction capacity: 55 mL; suction frequency: 30 s; suction duration: 3 s; and times of suction: 50. The aerosol collected on the Cambridge filter was collected and extracted by oscillation using an initial mobile phase.

### 3. Chromatographic condition of high performance liquid chromatograph used

Thermo Fisher Scientific Acclaim 120 C18 (4.6*250 mm, 5 µm) chromatographic column was used. The Method for Measuring Substance Related to AMB in the 2020 edition *Chinese Pharmacopoeia* was referred to, with a mobile phase: acetonitrile : 0.01 mol/L diammonium hydrogen phosphate solution (adjusted to pH 7.0 with phosphoric acid) = 50:50; flow rate: 1.0 mL/min; detection wavelength: 248 nm; and column temperature: 30°C.

### 4. Test of conversion rate of ambroxol hydrochloride atomized to generate aerosol

### 4.1 Preparation of solution in blank control group and liquid preparation of ambroxol hydrochloride, and collection of ambroxol hydrochloride aerosol

Blank control: initial mobile phase.

Ambroxol hydrochloride atomization solution: an appropriate amount of atomization solution was taken and diluted with the initial mobile phase.

Extraction of ambroxol hydrochloride aerosol: a Cambridge filter collected during aerosol collection was taken and extracted by oscillation using the initial mobile phase.

### 4.2 Calculation method

The atomization conversion rate was calculated using a formula: A=w1/w2*100%
A=atomization conversion rate
w1=mass percentage of the ambroxol hydrochloride in the aerosol
w2=mass percentage of the ambroxol hydrochloride in the liquid preparation

The results of the test of the atomization conversion rate of the liquid preparation of ambroxol hydrochloride with a mass percentage of 1% are shown in Table 2 below.

**Table 2 Test results of atomization of 1% ambroxol hydrochloride**

| Sample | Weight of aerosol /g | Concentration of sample/g·mL⁻¹ | w/% | A/% |
|---|---|---|---|---|
| Aerosol | 0.2489 | 0.024 | 0.987 | 98.50 |
| Liquid preparation | | 0.035 | 1.002 | |

| | | | | |
|---|---|---|---|---|
| Note: w represents the mass percentage of the ambroxol hydrochloride in the aerosol/liquid preparation; and A represents the atomization conversion rate. | | | | |

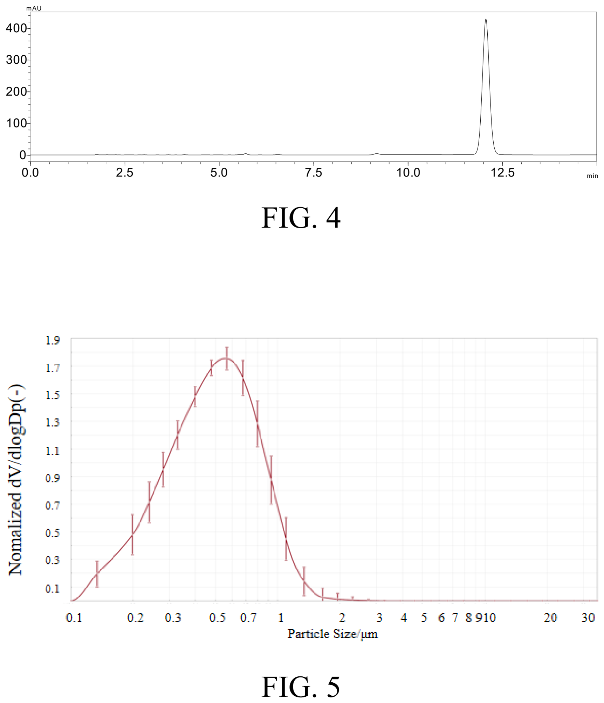

FIG. 3 shows a chromatogram of the liquid preparation of ambroxol hydrochloride, and FIG. 4 shows a chromatogram of the aerosol generated by atomizing the ambroxol hydrochloride. Comparing FIG. 3 and FIG. 4, it can be seen that there is no significant change in the peak shape of the chromatographic peak of the atomized ambroxol hydrochloride, and there are substantially no excess peaks in FIG. 4 compared to FIG. 3, indicating that the composition of the ambroxol hydrochloride is stable after the ambroxol hydrochloride is atomized, and the ambroxol hydrochloride may not undergo cracking of substances due to atomization which may affect the efficacy.

The atomization conversion rate of the 1% ambroxol hydrochloride measured by the above calculation method is 98.5%.

Embodiment IV of the present application takes a liquid preparation of ambroxol hydrochloride with a weight percentage of 0.5% as an example, and analyzes the conversion uniformity of the aerosol by suctioning repeatedly and analyzing the weight of ambroxol hydrochloride in the aerosol suctioned each time.

The mass percentages of the components in the liquid preparation are as follows: ambroxol hydrochloride: 0.5%; vegetable glycerin: 50%; 1,2-propylene glycol: 20%; and water: 29.5%.

The above liquid preparation in this embodiment was added to an electronic atomizer, a Cerulean smoking machine was used for suction, and the aerosol was collected using a single Cambridge filter. The Cambridge filter was collected every 25 times of suction (250 times of suction in total). The measured aerosol conversion rates are shown in Table 3 below.

**Table 3 Test results of atomization conversion rates of ambroxol hydrochloride**

| No. | Times of suction | Weight of aerosol/g | A/% |
|---|---|---|---|
| 1 | 25 | 0.1276 | 96.61 |
| 2 | 50 | 0.1228 | 95.49 |
| 3 | 75 | 0.1300 | 101.73 |
| 4 | 100 | 0.1314 | 97.05 |
| 5 | 125 | 0.1240 | 98.90 |
| 6 | 150 | 0.1332 | 101.84 |
| 7 | 175 | 0.1288 | 100.66 |
| 8 | 200 | 0.1367 | 100.23 |
| 9 | 225 | 0.1319 | 100.29 |
| 10 | 250 | 0.1249 | 96.93 |

| | | | |
|---|---|---|---|
| Note: A represents the atomization conversion rate. | | | |

From Table 3, the difference in weight of the ambroxol hydrochloride in the aerosols collected from every 25 times of suction is small, and the conversion rates are all greater than 90%. Therefore, the conversion rate of the ambroxol hydrochloride in the liquid preparation is relatively stable and has good uniformity. It should be noted that there are cases in the above table where the atomization conversion rate is greater than 100%, mainly due to the influence of the accuracy of the testing method, the instrument precision, and the like which result in numerical deviations.

It should be noted that most of electronic atomizers currently used have around 500 times of suction. The above experiment tested the stability of the ambroxol hydrochloride in weight in the aerosols collected every 25 times of suction and found that the uniformity is good. Therefore, it is fully proved that the relief effect on discomfort symptoms is uniform when a user repeatedly inhales the aerosol containing the ambroxol hydrochloride, and the ambroxol hydrochloride is suitable for use in electronic atomizers for atomization inhalation.

Embodiment V of the present application uses a Sympatec HELOS BR laser particle size analyzer from Germany to measure the particle size distribution of an ambroxol hydrochloride aerosol, and a smoking machine made by Shenzhen First Union Technology Co., Ltd.

The testing method used is as follows:
An R1 lens (test range 0.1-35 µm) was selected to collect particle size ranges with equal logarithmic intervals. There are 10 channels for actually measuring signals in a submicron range, with a high particle size resolution. Suction mode of an electronic atomizer: suction capacity: 55 mL; suction duration: 3 s; and suction interval: 30 s. A square wave suction curve was used. 3 parallel tests were conducted for each sample, with 3 times of suction per test, and the test results were averaged. One time slice was taken every 100 milliseconds, and a total of 30 slices were taken in 3 seconds of suction. The flow rate was 18.3 L/min.

**Table 4 Test results of particle size of ambroxol hydrochloride**

| Sample | Dv10/µm | Dv50/µm | Dv90/µm | Span |
|---|---|---|---|---|
| Ambroxol hydrochloride | 0.27 | 0.53 | 0.93 | 1.26 |

The results in Table 4 show that the median particle size of the ambroxol hydrochloride aerosol is 0.53 µm through testing. The particle size distribution of the ambroxol hydrochloride aerosol is shown in FIG. 5. The aerosol exhibits unimodal approximate logarithmic normal distribution, and the particle size of the aerosol is mainly distributed in a range of 0.2-1 µm, with a few aerosol particles in a range of 1-3 µm.

It should be noted that the particle size of the aerosol of ambroxol hydrochloride is small, so the taste of the aerosol is more delicate.

Embodiment V of the present application provides a liquid preparation containing ambroxol hydrochloride suitable for atomization using an electronic atomizer. The weight percentage of the ambroxol hydrochloride in the liquid preparation is 0.01-2%; or the weight percentage of the ambroxol hydrochloride in the liquid preparation is 0.1-1%.

Ambroxol hydrochloride is administered as a tablet, with a daily dosage limit of no more than 4 mg for adults. Therefore, by combining the capacity of an aerosol in each puff of the electronic atomizer and the number of inhalations of a user per day, the content of ambroxol hydrochloride is controlled within 2%, to allow the user to inhale multiple times within a day to alleviate symptoms of discomfort with rhinitis or asthma.

Further, by testing the atomization effect of the liquid preparation of ambroxol hydrochloride, it is found that when the weight percentage of the ambroxol hydrochloride in the liquid preparation is controlled at around 0.1-1%, the conversion rate of the ambroxol hydrochloride in the liquid preparation into the aerosol is substantially 90% or higher, even close to 100%. Therefore, controlling the weight percentage of the ambroxol hydrochloride in the liquid preparation within 0.1-1% is beneficial for improving the conversion rate of the ambroxol hydrochloride.

In some embodiments, the solvent includes at least one of propylene glycol, vegetable glycerin, water, or ethanol.

In some embodiments, the weight percentage of the propylene glycol in the preparation composition for inhalation is 20-80%.

In some embodiments, the weight percentage of the vegetable glycerin in the preparation composition for inhalation is 30-70%.

The above propylene glycol may be 1,2-propylene glycol or 1,3-propylene glycol. Propylene glycol and vegetable glycerin, as common solvents in electronic atomizers, have excellent performance in increasing the aerosol amount and controlling the fluidity of a liquid preparation. Therefore, propylene glycol and vegetable glycerin are the main solvent components. Also, an appropriate amount of water or ethanol may be added as a solvent as the weight percentage of the ambroxol hydrochloride in the liquid preparation increases, thereby increasing the solubility of the ambroxol hydrochloride in the liquid preparation.

In some embodiments, the atomization conversion rate of the ambroxol hydrochloride is not less than 90%; or the atomization conversion rate of the ambroxol hydrochloride is not less than 80%; or the atomization conversion rate of the ambroxol hydrochloride is not less than 70%.

By the atomization testing method according to the embodiments of the present application, it is found that the liquid preparation containing a certain amount of ambroxol hydrochloride has a atomization conversion rate of not less than 90%, and the weight percentage content of the ambroxol hydrochloride in the liquid preparation is roughly set to be 1% or less. When the content of the ambroxol hydrochloride in the liquid preparation is adjusted to be in a certain range, the conversion rate of the ambroxol hydrochloride is not less than 80%. When the weight percentage content of the ambroxol hydrochloride in the liquid preparation is further adjusted to be in other ranges, or a different electronic atomizer is used, the conversion rate of the ambroxol hydrochloride is not less than 70%. The different electronic atomizer is configured with a different heating power or uses a different atomization core component.

In some embodiments, the liquid preparation further includes at least one of an edible essence, a sweetener, or a cooling agent.

The above edible essence is used to increase the taste and aromatic flavor of the aerosol generated by atomizing the liquid preparation. The types of flavors of the edible essence include one or more of fruit flavor, flower flavor, mint flavor, and tea flavor.

The weight percentage of the above edible essence in the liquid preparation is in a range of roughly 0-10%, and the specific content may be set to be any value in the range of 0-10% according to the taste adjustment needs of the liquid preparation.

The above sweetener is used to enhance the sweetness of the aerosol generated by atomizing the liquid preparation. It should be noted that the ambroxol hydrochloride has a certain bitterness, so adding an appropriate amount of sweetener to the liquid preparation can help enhance the taste. The sweetener may be selected from the group consisting of one or more of neotame, cyclamate, sucralose, aspartame, stevioside, and acesulfame K.

The weight percentage of the above sweetener in the liquid preparation is in a range of roughly 0-10%, and the specific content may be set to be any value in the range of 0-10% according to the taste adjustment needs of the liquid preparation.

The above cooling agent is used to increase the cooling effect of the above aerosol generated by atomizing the liquid preparation, thereby enhancing a user's pleasure in inhaling the aerosol. Suitable cooling agents for use in the above liquid preparation may be one or more of menthol, menthone, isomenthone, WS-23 (2-isopropyl-N,2,3-trimethylbutanamide), WS-3 (N-ethyl-p-menthyl-3-carboxamide), and WS-5 (N-(ethoxycarbonylmethyl)-p-alkyl-3-carboxamide).

The weight percentage of the above cooling agent in the liquid preparation is in a range of roughly 0-10%, and the specific content may be set to be any value in the range of 0-10% according to the taste adjustment needs of the liquid preparation.

It should be noted that the inventor tested the atomization performance of more than 20 drug reagents and found that most of the drug reagents are not suitable for atomization inhalation using an electronic atomizer. For example, the atomization conversion rate of clenbuterol hydrochloride is less than 10%, the atomization conversion rate of disodium cromoglycate is less than 15%, and the atomization conversion rate of terbutaline sulfate is less than 50%. Due to low conversion rates, the content of functional components in an aerosol is low, making it difficult to achieve the effect of relieving discomfort symptoms even if a user inhales multiple times. The liquid preparation containing ambroxol hydrochloride according to the embodiments of the present application, when atomized using an electronic atomizer, has a high conversion rate of the functional components into an aerosol and good uniformity, resulting in unexpected technical effects. Therefore, the liquid preparation is suitable to be atomized using an electronic atomizer to generate an aerosol for a user to inhale to alleviate discomfort symptoms.

Embodiment VI of the present application provides a cartridge, used for an electronic atomizer, and the cartridge stores the liquid preparation containing ambroxol hydrochloride.

The above cartridge may be provided as a closed container that may be sold independently and assembled on an electronic atomizer. The liquid preparation in the cartridge may flow towards a atomization core component on the electronic atomizer and be atomized by the atomization core component to generate an aerosol.

The above cartridge may also be provided as an inseparable part of the electronic atomizer. The liquid preparation in the cartridge may flow towards a atomization core component on the electronic atomizer and be atomized by the atomization core component to generate an aerosol. As the liquid preparation in the cartridge is consumed, at least a part of the electronic atomizer may be discarded.

Embodiment VII of the present application provides an aerosol generating system, including the above liquid preparation and an electronic atomizer for atomizing the liquid preparation to generate an aerosol.

In some embodiments, the electronic atomizer includes a atomizer using a ceramic core or a atomizer using a cotton core.

The atomizer using a ceramic core includes a porous liquid guide body and a heating element combined on the porous liquid guide body. The porous liquid guide body is made of a porous material, and the porous material includes microporous ceramic, microporous glass ceramic, microporous glass, or metal foam. The heating element may be made of a metal material having appropriate impedance, a metal alloy, graphite, carbon, conductive ceramic, or a composite material of other ceramic materials and metal materials. Suitable metal or alloy materials include at least one of nickel, cobalt, zirconium, titanium, nickel alloys, cobalt alloys, zirconium alloys, titanium alloys, nickel chromium alloys, nickel iron alloys, iron chromium alloys, titanium alloys, iron manganese aluminum based alloys, or stainless steel etc.

The atomizer using a cotton core includes a liquid guide element and a heating element combined on the liquid guide element. The liquid guide element is made of fiber cotton, and the material for preparing the heating element in the atomizer using a cotton core is roughly the same as that for preparing the heating element in the atomizer using a ceramic core.

In addition to the atomization core component, the electronic atomizer includes a battery component for providing electric drive.

The electronic atomizer is preferably arranged as a portable device with a small volume, thereby facilitating a user to carry and inhale at any time to alleviate discomfort symptoms such as rhinitis or asthma.

Compared to an ultrasonic atomizer which generates water mist by breaking up the molecular structure of a liquid substrate through high-frequency oscillation of a mesh atomizing plate, an electronic atomizer brings a liquid substrate to come to the boiling point and generate an aerosol through an electric heating process. The particle size of most of the aerosol generated by the liquid preparation containing ambroxol hydrochloride through the electric heating process using the electronic atomizer is less than or equal to 1 µm, which is conducive to deep deposition of the aerosol in the bronchi and lungs, especially in the deep alveoli, and is suitable for drugs for administration in the lower respiratory tract and lungs. Combining the significant therapeutic effect of the ambroxol hydrochloride in the treatment of respiratory disease, the liquid preparation containing the ambroxol hydrochloride being atomized through electric heating using the electronic atomizer is more conducive to utilization of the therapeutic effect of the ambroxol hydrochloride. On the other hand, by atomizing the liquid preparation containing the ambroxol hydrochloride using the electronic atomizer, the generated ambroxol hydrochloride aerosol has less residue in the oral cavity and less irritation to the stomach, which is beneficial for improving user compliance. Further, the electronic atomizer is configured to an active inhalation mode, which activates a heating system through a user's active inhalation, and then the aerosol containing ambroxol hydrochloride generated by atomization through electric heating is inhaled, which can further enhance the user compliance.

It should be noted that exemplary embodiments of the present application are provided in the specification and the accompanying drawings, but are not limited to the embodiments described in the specification. Furthermore, for those of ordinary skills in the art, improvements or transformations can be made based on the above description, and all such improvements and transformations should fall within the protection scope of the claims attached to the present application.

## Claims

1. A liquid preparation for atomization inhalation, for use with an electronic atomizer, comprising:
ambroxol hydrochloride; and
a solvent, wherein
when the ambroxol hydrochloride in the liquid preparation is heated and atomized by the electronic atomizer to generate an aerosol, the atomization conversion rate of the ambroxol hydrochloride is not lower than 90%;
or the atomization conversion rate of the ambroxol hydrochloride is not lower than 80%;
or the atomization conversion rate of the ambroxol hydrochloride is not lower than 70%;
or the atomization conversion rate of the ambroxol hydrochloride is not lower than 60%.

2. The liquid preparation for atomization inhalation according to claim 1, wherein the weight percentage of the ambroxol hydrochloride in the liquid preparation is 0.01-2%; or the weight percentage of the ambroxol hydrochloride in the liquid preparation is 0.1-1%.

3. The liquid preparation for atomization inhalation according to claim 1, wherein the solvent comprises at least one of propylene glycol, vegetable glycerin, water, or ethanol.

4. The liquid preparation for atomization inhalation according to claim 3, wherein the weight percentage of the propylene glycol in the liquid preparation composition is 20-80%.

5. The liquid preparation for atomization inhalation according to claim 3, wherein the weight percentage of the vegetable glycerin in the liquid preparation composition is 30-70%.

6. The liquid preparation for atomization inhalation according to claim 3, wherein the weight percentage of the water in the liquid preparation is 10-50%.

7. The liquid preparation for atomization inhalation according to claim 1, wherein the particle size of the aerosol generated by atomizing the ambroxol hydrochloride in the liquid preparation using the electronic atomizer is in a range of 0.2-3 µm.

8. The liquid preparation for atomization inhalation according to claim 1, wherein the liquid preparation further comprises at least one of an edible essence, a sweetener, or a cooling agent.

9. A cartridge, for use with an electronic atomizer, wherein the cartridge stores the liquid preparation for atomization inhalation according to any one of claims 1-8.

10. An aerosol generating system, comprising the liquid preparation for atomization inhalation according to any one of claims 1-8, and an electronic atomizer configured to heat and atomize the liquid preparation to generate an aerosol.

11. The aerosol generating system according to claim 10, wherein the electronic atomizer comprises a atomizer using a ceramic core or a atomizer using a cotton core.
